# EUROPEAN PATENT APPLICATION

(11) **EP 2 940 614 A1**
(43) Date of publication of application: **04.11.2015**
(21) Application number: 15165894.5
(22) Date of filing: 30.04.2015
(51) Int. Cl.: G06F 19/00

(54) **MEDICATION DISPENSING APPARATUS**

(30) Priority: 30.04.2014 KR 20140052174
(71) Applicant: JVM Co., Ltd., Daegu 704-946 (KR)
(72) Inventor: Kim, Jun Ho, 706-819 Daegu (KR)
(74) Representative: Dantz, Jan Henning

(57) **Abstract**

There is provided a medication dispensing apparatus dispensing a medication from a medication storing unit on a basis of medication dispensing information of which a formal sequence of medication dispensing is determined. The medication dispensing apparatus includes an interface unit connected to a first medication storage unit holding a first medication, a communication unit receiving the medication dispensing information, and a controller dispensing a medication in the formal sequence of the medication dispensing information received through the communication unit and when next time medication dispensing information is the first medication dispensing information including the first medication, taking a sequence of medication dispensing information remaining by omitting the first medication dispensing information as a real sequence of medication dispensing to dispense the medication in the real sequence.

## Description

### CROSS-REFERENCE TO RELATED PATENT APPLICATION

This application claims the benefit of Korean Patent Application No. 10-2014-0052174, filed on April 30, 2014, in the Korean Intellectual Property Office, the disclosure of which is incorporated herein in its entirety by reference.

### BACKGROUND OF THE INVENTION

### 1. Field of the Invention

The present invention relates to a medication dispensing apparatus, and more particularly to a medication dispensing apparatus for improving a pharmacy's efficiency of medication on the basis of medication dispensing information.

### 2. Description of the Related Art

Typically, a medication dispensing apparatus is a kind of medication packaging apparatus automatically or manually dispensing medications from a plurality of medication storage units in which the medications are stored according to a sequence of medication dispensing information such as prescription and then allowing the medications to be packaged in a packaging unit such as medication wrapping paper.

Here, the medication storage units disposed in the medication dispensing apparatus are largely classified into two types and one of them is a general medication storage unit in which medications are plentifully stored at a predetermined position in the medication dispensing apparatus to enable automatic distribution as necessary, and the other is a special medication storage unit such as a tray on which medications are required to be manually distributed every time needed.

In other words, it is reality that medications held on the tray are medications such as drugs needing management and supervision by a manager, should be dispensed to the medication dispensing apparatus through the tray only when the medications are needed to be dispensed according to the medication dispensing information, and may not be stored in the medication dispensing apparatus when the medication dispensing apparatus does not operate.

Furthermore, medications to be packaged in one piece of medication wrapping paper on the basis of the medication dispensing information may include all medications stored in the general medication storage unit and the special medication storage unit, and the medication dispensing apparatus sequentially packages medications in the medication wrapping paper in order of medication dispensing information such as received prescription.

Herein, in a process for packaging medications in the medication wrapping paper in order of the medication dispensing information, when there is no medications stored in the special medication storage unit, an operation of a typical medication dispensing apparatus is stopped until the medications are supplemented in the special medication storage unit, namely, maintains a standby state until the medications are prepared.

In this case, for a time when the medications are supplemented in the special medication storage unit, a pharmacy step such as wrapping medications in the medication wrapping paper is not proceeded and accordingly a pharmacy's efficiency is lowered as much as that.

Accordingly, researches for maximizing a pharmacy's efficiency are urgent for the medication dispensing apparatus holding the general and special medication storage units.

### SUMMARY OF THE INVENTION

The present invention provides a medication dispensing apparatus dispensing medication in order of medication dispensing information such as prescription but enabling the order of the medication dispensing information to be variable to improve a pharmacy's efficiency in dispensing medications and packaging medications in medication wrapping paper.

The technical objects of the present invention are not limited to those described above, and it will be apparent to those of ordinary skill in the art from the following description that the present invention includes other technical objects not specifically mentioned herein.

According to an aspect of the present invention, a medication dispensing apparatus dispenses a medication from a medication storing unit on a basis of medication dispensing information of which a formal sequence of medication dispensing is determined. The medication dispensing apparatus includes: an interface unit connected to a first medication storage unit holding a first medication; a communication unit receiving the medication dispensing information; and a controller dispensing a medication in the formal sequence of the medication dispensing information received through the communication unit and when next time medication dispensing information is the first medication dispensing information including the first medication, taking a sequence of medication dispensing information remaining by omitting the first medication dispensing information as a real sequence of medication dispensing to dispense the medication in the real sequence.

When the first medication storage unit holding the first medication is connected to the interface unit, the controller may change the real sequence of medication dispensing for prioritizing the first medication dispensing information and dispenses the medication in the changed real sequence.

When the first medication dispensing information includes 1-1 medication dispensing information and 1-2 medication dispensing information, the controller may dispense the first medication on a basis of the 1-1 medication dispensing information and then sequentially dispenses the first medication on a basis of the 1-2 medication dispensing information.

When an amount of the first medication remaining in the first medication storage unit is smaller than that included in the 1-2 medication dispensing information, the controller may dispense the medication in a sequence of medication dispensing information remaining after the 1-2 medication dispensing information is omitted from medication dispensing information that medication dispensing is necessary.

When medication dispensing information additionally received through the communication unit includes the first medication, the controller may dispense the first medication on the basis of the first medication dispensing information and then sequentially dispenses the first medication on the basis of the additionally received medication dispensing information.

When the first medication storing unit holding the first medication is connected to the interface unit while the medication is dispensed in the real sequence, the controller may make the first medication dispensing information come directly behind medication dispensing information corresponding to a medication currently dispensed and dispenses the medication.

### BRIEF DESCRIPTION OF THE DRAWINGS

The above and other features and advantages of the present invention will become more apparent by describing in detail exemplary embodiments thereof with reference to the attached drawings in which:
Fig. 1 is a schematic perspective view illustrating a medication dispensing apparatus according to an embodiment of the present invention;
Fig. 2 is a block configuration diagram illustrating a medication dispensing apparatus according to an embodiment of the present invention;
Fig. 3 is a flowchart for explaining an operation sequence of a medication dispensing apparatus according to an embodiment of the present invention;
Fig. 4 is a flowchart for explaining a method of determining a medication dispensing sequence of a medication dispensing apparatus according to an embodiment of the present invention;
Fig. 5 is a flowchart for explaining a method of determining a real sequence of a medication dispensing sequence of a medication dispensing apparatus according to an embodiment of the present invention; and
Fig. 6 is a flowchart for explaining a method of determining a medication dispensing sequence of a medication dispensing apparatus according to another embodiment of the present invention.

### DETAILED DESCRIPTION OF THE INVENTION

Hereinafter, preferred embodiments will be described in more detail with reference to the accompanying drawings. Moreover, detailed descriptions of well-known functions or configurations will be omitted in order not to unnecessarily obscure the focus of the present invention. However technical concepts of the invention are not limited within the proposed embodiments. On the contrary, by addition of other constituting elements, change or deletion of the constituting elements from the present invention, another retrogressive invention or other embodiments that fall within the scope of the present invention can be easily suggested.

Also, the same or similar reference numerals provided in each drawing denote the same or similar components having the same functions in the scope of the same idea.

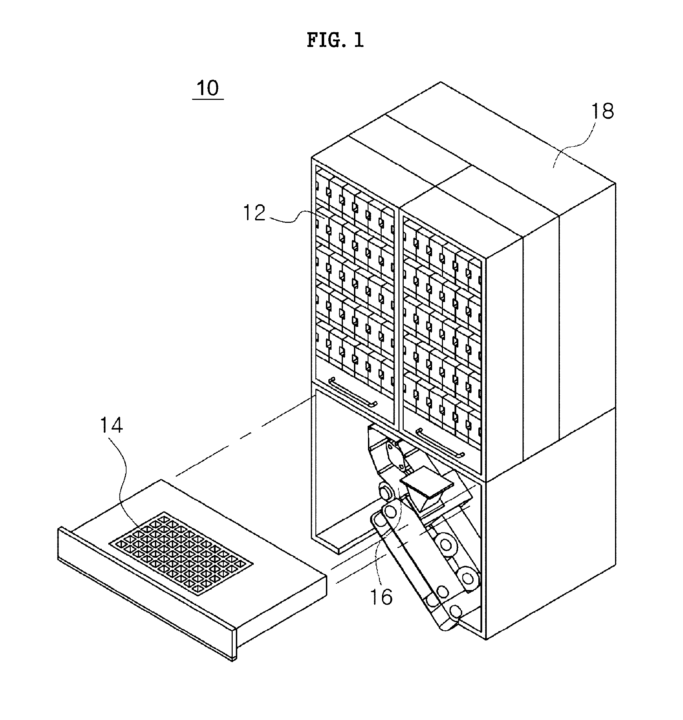

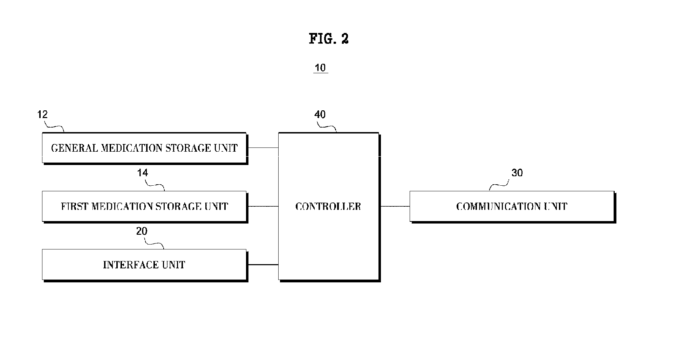

Fig. 1 is a schematic perspective view illustrating a medication dispensing apparatus according to an embodiment of the present invention, and Fig. 2 is a block configuration diagram illustrating a medication dispensing apparatus according to an embodiment of the present invention.

Referring to Figs. 1 and 2, a medication dispensing apparatus 10 according to an embodiment of the present invention may include a general medication storage unit 12 storing general medications, a first medication storage unit 14 storing a first medication, a communication unit 30 receiving medication dispensing information, and a controller 40 controlling medication dispensing.

Here, the medication dispensing apparatus 10 may be an apparatus for dispensing medications from the general medication storage unit 12 and the first medication storage unit 14 on the basis of medication dispensing information of which a formal sequence of medication dispensing is determined.

In addition, the medication dispensing information may be information including information on a medication to be taken by a patient, for example, a prescription for the patient.

The medication dispensing information may include personal information on a patient, information on a type and amount of medication to be taken, information on the number of days to take a medication and information on illness.

The general medication storage unit 12 may be mounted in plurality in a main body 18 providing an appearance of the medication dispensing apparatus 10 and medications stored inside may be general medications storable plentifully in the main body 18 even when the medication dispensing apparatus 10 is not in operation.

Here, the general medication storage unit 12 storing specific general medications may be at least one, and the controller 40 may select a specific general medication storage unit 12 on the basis of a time taken to dispense a medication, a movement distance of the dispensed medication, an inventory amount, when dispensing the specific general medication.

On the contrary, the first medication storage unit 14 may be a tray configured with pluralities of rows and columns, a held medication therein may be a medication that history management by a manager is very important, unlike medications stored in the general medication storage unit 12, for example, a drug. The stored medications therein are limited in number and have a characteristic of being distributed manually by the reason of management, every time dispensing is necessary.

The first medication storage unit 14 may be detachable from the main body 18 or mounted in the main body by a withdrawable interface unit 20 and the interface unit 20 may recognize identification information on the first medication storage unit 14.

The identification information may be unique identification information for distinguishing different first medication storage units 14 from each other, and information including a type or amount of the first medication.

Here, when a recognition method of the identification is a tag method based on a near field communication (NFC) technique, the interface unit 20 may be implemented with a reader based on the NFC technique, and when being a tag method based on a radio frequency identification (RFID) technique, the interface unit 20 may be implemented with a reader based on the RFID technique.

In addition, when a recognition method of the identification information is a memory chip method in a plug method, the interface unit 20 may be implemented with a port designed to be connected to the memory chip.

However, the identification information recognition method for the first medication storage unit 14 by the interface unit 20 is not limited to the above-described methods.

In addition, the interface unit 20 may be just a mount space where the first medication storage unit 14 is mounted and be a medium structure allowing the first medication storage unit 14 to be mounted in the main body 18.

In this case, whether the first medication storage unit 14 is mounted in the interface unit 20 may be recognized by using a known method such as use of a sensor.

Furthermore, the medication dispensing apparatus 10 may include a packaging unit 16 allowing at least one of the general medication and first medication to be dispensed from at least one of the general medication storage unit 12 and the first medication storage unit 14 and packaged in medication wrapping paper on the basis of the medication dispensing information, and finally realize pharmacy of medications packaged based on the medication dispensing information.

The communication unit 30 may be a component disposed in the main body 18 and enabling data communication to an external device, and in detail, may receive medication dispensing information that is a kind of prescription for a patient through a server or a user terminal equipped in a hospital, ward, or pharmacy.

The communication unit 30 may be realized with a wired/wireless communication interface. In a case of wired communication, the communication unit 30 may use a wired cable, and in a case of wireless communication, use wireless LAN (WLAN, WiFi), wireless broadband (Wibro), world interoperability for microwave access (Wimax), high speed downlink packet access (HSDPA) or the like.

The communication unit 30 may include a short range communication module and use Bluetooth, RFID, infrared data association (IrDA), ultra wideband (UWB), ZigBee, WiHD, WiGig, or the like.

The controller 40 may determine a medication dispensing sequence based on the medication dispensing information to dispense medications, and a detailed method thereof will be described as follows.

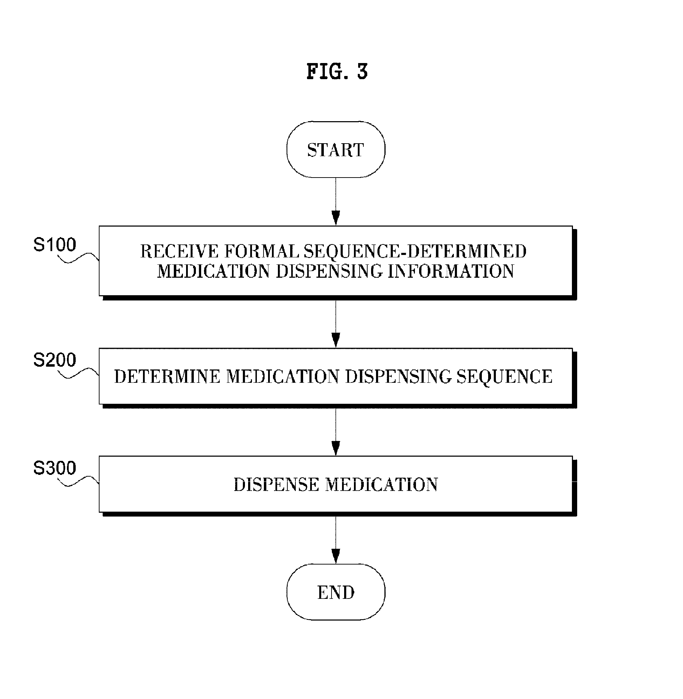

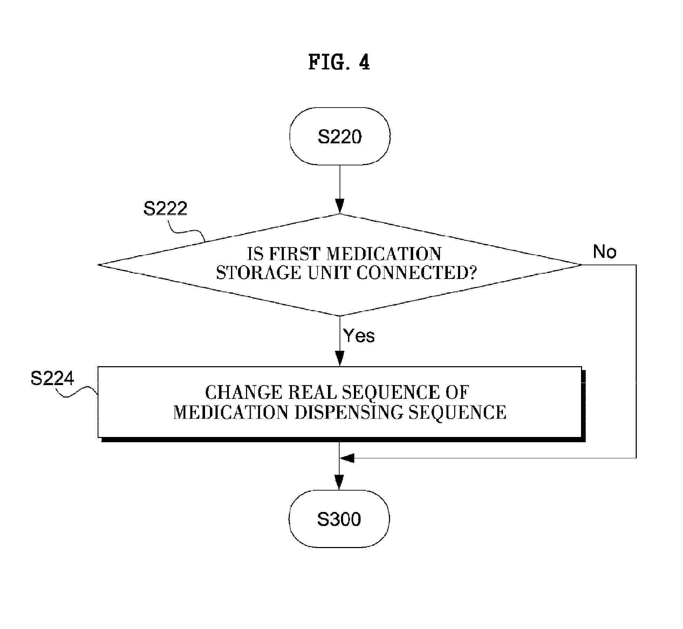

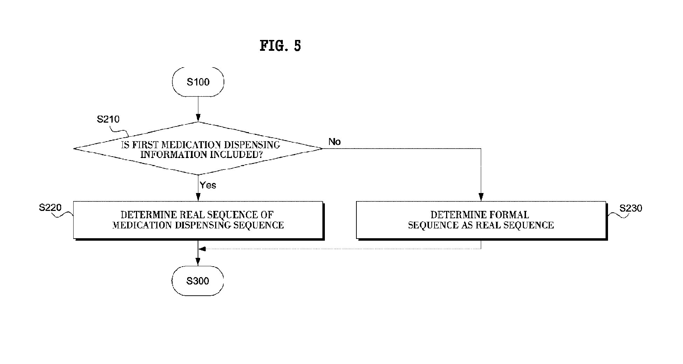

Fig. 3 is a flowchart for explaining an operation sequence of the medication dispensing apparatus according to an embodiment of the present invention, Fig. 4 is a flowchart for explaining a method of determining a medication dispensing sequence of the medication dispensing apparatus according to an embodiment of the present invention, and Fig. 5 is a flowchart for explaining a method of determining a real sequence of the medication dispensing of a medication dispending apparatus according to an embodiment of the present invention.

Referring to Fig. 3, an operation sequence of the medication dispensing apparatus 10 according to an embodiment may include an operation S100 of receiving medication dispensing information that a formal sequence is determined, an operation S200 of determining a medication dispensing sequence S200, and an operation of dispensing medications S300.

Hereinafter, each operation will be described in detail with reference to Figs. 4 and 5.

As illustrated in Fig. 4, the controller 40 may receive medication dispensing information that is a kind of prescription for a patient from a server or a user terminal equipped in a hospital, ward, or pharmacy, through the communication unit 30 (operation S100).

A formal sequence of medication dispensing of the received medication dispensing information may be determined. For example, when the communication unit 30 receives medication dispensing information A, medication dispensing information B, medication dispensing information C, medication dispensing information D, medication dispensing information E, medication dispensing information F, and medication dispensing information G, a formal sequence of medication dispensing may be that the medication dispensing information A is the first, the medication dispensing information B is the second, the medication dispensing information C is the third, the medication dispensing information D is the fourth, the medication dispensing information E is the fifth, the medication dispensing information F is the sixth, and the medication dispensing information G is the seventh.

In other words, the medication dispensing apparatus 10 allows medications corresponding to each piece of medication dispending information to be dispensed and packaged in medication wrapping paper.

In addition, the number of pieces of medication wrapping paper corresponding to one piece of medication dispensing information may be diversely changed according to the number of medication taking days or the like.

When medication dispensing information of which a formal sequence is determined is received through the communication unit 30, the controller 40 may determine whether the received medication dispensing information includes the first medication dispensing information (operation S210).

As described in relation to Figs. 1 and 2, the first medication is a medication that history management by a manager is very important and may be a medication stored in the first medication storage unit 14 such as a kind of tray.

Here, the controller 40 may change the medication dispensing sequence when the first medication dispensing information is included in the medication dispensing information received through the communication unit 30.

In detail, the controller 40 may determine, as a real sequence of medication dispensing, a sequence of medication dispensing information remaining by excluding the first medication dispensing information from the medication dispensing information on the basis of the first medication dispensing information including the first medication in the medication dispensing information received through the communication unit 30 (operation S220), and may dispense the medications according to the determined real sequence.

For example, when the medication dispensing information is the first medication dispensing information including the first medication in the medication dispensing information A to the medication dispensing information G received through the communication unit 30, the controller 40 may determine the real sequence of medication dispensing as a sequence of the medication dispensing information A, the medication dispensing information C, the medication dispensing information D, the medication dispensing information E, the medication dispensing information F, and the medication dispensing information G.

Since the first medication storage unit 14 holds the first medication of which history management by a manager is very important and has no choice but to manually distribute it every time needed, unlike the general medication storage unit 12, a case where the first medication storage unit 14 is not mounted or connected to the interface unit 20 of the medication dispensing apparatus 10 may occur. In this case, there may be a limitation that an operation of the medication dispensing apparatus 10 should be stopped until the first medication storage unit 14 is mounted in the interface unit 20 at the time of the medication dispensing information B following the medication dispensing information A, when the medication is dispensed in an original formal sequence.

Since this brings to lower the medication dispensing efficiency and pharmacy's efficiency such as packaging medications in the medication wrapping paper, in the present invention, the pharmacy's efficiency may be improved by excluding, by the controller 40, the medication dispensing information including the first medication dispensing information from the medication dispensing sequence.

Here, when the first medication dispensing information including the first medication is in plurality in the medication dispensing information A to medication dispensing information G received through the communication unit 30, the controller 40 may dispense medications by taking a sequence of medication dispensing information remaining by excluding the plural pieces of the first medication dispensing information as the real sequence of medication dispensing.

Furthermore, when determining that the first medication dispensing information including the first medication is not included in the medication dispensing information A to medication dispensing information G received through the communication unit 30, the controller 30 may determine a formal sequence of the medication dispensing information received through the communication unit 30 as a real sequence (operation S230) and dispense the medications in the real sequence (operation S300).

Referring to Fig. 5, when determining that the first medication dispensing information including the first medication is included in the medication dispensing information received through the communication unit 30 in operation S220, the controller 40 may determine whether the first medication storage unit 14 holding the first medication is mounted in the interface unit 20 (operation S222).

As a determination result, when the first medication storage unit 14 is mounted in the interface unit 20, the real sequence of the medication dispensing may be changed (operation S224).

In detail, when the first medication storage unit 14 is connected to the interface unit 20, the controller 40 changes the real sequence of medication dispensing in order that the first medication dispensing information determined in operation S210 comes first (operation S224) and dispense the medications in the changed real sequence (operation S300).

For example, when the medication dispensing information B in the medication dispensing information A to medication dispensing information G received through the communication unit 20 is the first medication dispensing information including the first medication, the controller 40 may determine the real sequence of medication dispensing as a sequence of the medication dispensing information A, medication dispensing information C, medication dispensing information D, medication dispensing information E, medication dispensing information F, and medication dispensing information G through operation S220. However, when the first medication storage unit 14 is connected to the interface unit 20 in operation S222, the controller 40 may determine again the medication dispensing sequence as a sequence of the medication dispensing information B, medication dispensing information A, medication dispensing information C, medication dispensing information D, medication dispensing information E, medication dispensing information F, and medication dispensing information G.

When the first medication storage unit 14 is connected to the interface unit 20, an operation of the medication dispensing apparatus 10, which is necessary for supplementing the first medication at the time of the first medication dispense, may be prevented from being stopped in advance by preferentially dispensing the medication dispensing information including the first medication (i.e., the medication dispensing information B) that history management thereof by a manager is very important than other medication dispensing information (medication dispensing information A, C to G) to realize supplement of the first medication to the first medication storage unit 14 for a time to dispense the medications according to the other medication dispensing information (i.e., medication dispensing information A, C to G), and to overcome a limit of space where the first medication storage unit 14 may hold the first medication

Furthermore, when the first medication dispensing information including the first medication in the medication dispensing information A to G received through the communication unit 30 is in plurality and the first medication storage unit 14 is connected to the interface unit 20, the controller 40 may dispense the first medication on the basis of any one piece of first medication dispensing information and then sequentially dispense the first medication on the basis of another piece of first medication dispensing information.

However, after the first medication is dispensed based on the any one piece of first medication dispensing information, when an amount of the first medication remaining in the first medication storage unit 14 is smaller than that included in the other piece of first medication dispensing information, the controller 40 may dispense medications in a formal sequence of the medication dispensing information remaining after the other first medication dispensing information is excluded from medication dispensing information that medication dispensing is necessary.

For example, when the medication dispensing information B and D in the medication dispensing information A to medication dispensing information G received through the communication unit 20 are the first medication dispensing information including the first medication, the controller 40 may determine the real sequence of medication dispensing as a sequence of the medication dispensing information A, medication dispensing information C, medication dispensing information E, medication dispensing information F, and medication dispensing information G through operation S220. However, when determining that the first medication storage unit 14 is connected to the interface unit 20 in operation S222, the controller 40 may determine again the medication dispensing sequence as a sequence of the medication dispensing information B, medication dispensing information D, medication dispensing information A, medication dispensing information C, medication dispensing information E, medication dispensing information F, and medication dispensing information G.

However, when an amount of the first medication remaining after the first medication is dispensed from the first medication storage unit 14 according to the medication dispensing information B is smaller than that included in the medication dispensing information D, the controller 40 may dispense medications in a sequence of the medication dispensing information B, medication dispensing information A, medication dispensing information C, medication dispensing information E, medication dispensing information F, and medication dispensing information G.

When determining that the first medication storage unit 14 is not connected to the interface unit 20 in operation S222, the controller 40 may dispense medications in the real sequence determined in operation S220 (operation S300).

Furthermore, the controller 40 may dispense medications in the formal sequence of the medication dispensing information received through the communication unit 30, and when the next time medication dispensing information is the first medication dispensing information including the first medication, may dispense the medications in a real sequence by taking, as the real sequence, a sequence of remaining medication dispensing information from which the first medication dispensing information is omitted.

For example, when the communication unit 30 receives the medication dispensing information A, medication dispensing information B, medication dispensing information C, medication dispensing information D, medication dispensing information E, medication dispensing information F, and medication dispensing information G, the controller 40 may sequentially dispense medications on the basis of the medication dispensing information A to medication dispensing information G and determine whether the next time medication dispensing information is the first medication dispensing information including the first medication while dispensing medications on the basis of any one piece of the medication dispensing information.

When the medication dispensing information C in the medication dispensing information A to medication dispensing information G is the first medication dispensing information including the first medication, the controller 40 may determine whether the first medication is included in the medication dispensing information B that is the next time medication dispensing information during medication dispensing according to the medication dispensing information A in a formal sequence, and since the medication dispensing information B does not include the first medication, when the medication dispensing is completed according to the medication dispensing information A in the formal sequence, the controller 40 may dispense medications according to the medication dispensing information B.

Here, while the medications are dispensed according to the medication dispensing information B, the controller 40 may determine whether the first medication is included in the medication dispensing information C that is the next time medication dispensing information. Since the medication dispensing information C is the first medication dispensing information including the first medication, the controller 40 may omit the medication dispensing information C from the dispensing sequence.

In other words, the real sequence of medication dispensing may be a sequence of the medication dispensing information A, medication dispensing information B, medication dispensing information D, medication dispensing information E, medication dispensing information F, and medication dispensing information G.

Here, controller 40 may determine whether the first medication storage unit 14 holding the first medication is connected to the interface unit 20, and as the determination result, when determined to be connected, the controller 40 may change the real sequence of the medication dispensing to dispense medications in the changed real sequence in order for the first medication dispensing information to be prioritized.

For example, when the medication dispensing information C among the medication dispensing information A to medication dispensing information G is the first medication dispensing information including the first medication, the controller 40 may determine whether the first medication is included in the medication dispensing information B that is the next time medication dispensing information during medication dispensing according to the medication dispensing information A in a formal sequence. Since the medication dispensing information B does not include the first medication, when the medication dispensing is completed according to the medication dispensing information A in the formal sequence, the controller 40 may dispense medications according to the medication dispensing information B.

While the medications are dispensed according to the medication dispensing information B, the controller 40 may determine whether the first medication is included in the medication dispensing information C that is the next time medication dispensing information. Since the medication dispensing information C is the first medication dispensing information including the first medication, the controller 40 may omit the medication dispensing information C from the dispensing sequence.

In addition, when the medication dispensing is completed according to the medication dispensing information B, the controller 40 may dispense medications according to the medication dispensing information D.

Here, the controller 40 may determine whether the first medication storage unit 14 holding the first medication is connected to the interface unit 20. As the determination result, when determining to be connected, the controller 40 may prioritize the medication dispensing information C that has been omitted to dispense medications according to the medication dispensing information D and then dispense the first medication according to the medication dispensing information C.

Then medications are dispensed in a sequence of the medication dispensing information E to medication dispensing information G as the formal sequence.

Furthermore, when the first medication dispensing information including the first medication is in plurality, the controller 40 may dispense the first medication on the basis of any one piece of first medication dispensing information and then sequentially dispense the first medication on the basis of another piece of the first medication dispensing information.

For example, when the first medication dispensing information including the first medication in the medication dispensing information A to G received through the communication unit 30 is in plurality, and the first medication storage unit 14 is connected to the interface unit 20, the controller 40 may dispense the first medication on the basis of any one piece of first medication dispensing information and then sequentially dispense the first medication on the basis of another piece of first medication dispensing information.

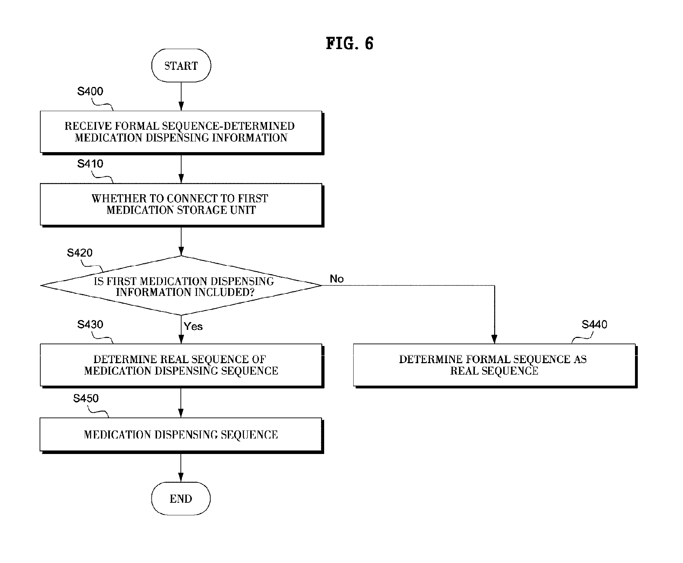

Fig. 6 is a flowchart for explaining a method of determining a medication dispensing sequence of a medication dispensing apparatus according to another embodiment of the present invention.

Referring to Fig. 6, a medication dispensing sequence according to another embodiment of a medication dispensing apparatus according to an embodiment of the present invention may include an operation S400 of receiving medication dispensing information of which a formal sequence is determined, an operation S410 of checking whether the first medication storage unit 14 is connected to the interface unit 20, an operation S420 of determining whether the first medication dispensing information including the first medication is included in medication dispensing information received through the communication unit 30, operations S430 and S440 of determining a real sequence, and an operation S450 of dispensing medications in the real sequence.

When the medication dispensing information of which a formal sequence is determined is received through the communication unit 30 (operation S400), the controller 40 may check whether the first medication storage unit 14 holding the first medication is mounted in the interface unit 20.

When it is checked that the first medication storage unit 14 is connected to the interface unit 20 (operation S410), the controller 40 may determine whether the first medication dispensing information including the first medication exists in at least one piece of the medication dispensing information received through the communication unit 30 (operation S420). As a determination result, when determined to exist, the controller 40 determine the real sequence of medication dispensing in order for the first medication dispensing information to be prioritized (operation S430), and dispense medications according to the determined real sequence (operation S450).

For example, when the medication dispensing information B in the medication dispensing information A to medication dispensing information G received through the communication unit 30 is the first medication dispensing information including the first medication and the first medication storage unit 14 is connected to the interface unit 20, the controller 40 determines whether the first medication dispensing information including the first medication exists in the medication dispensing information A to medication dispensing information G received through the communication unit 30.

When the medication dispensing information B is determined to be the first medication dispensing information, the controller 40 may determine a real sequence of medication dispensing allowing the medication dispensing information B to be preferred by another piece of medication dispensing information, and dispense medications according to the determined real sequence.

Finally, the real sequence of medication dispensing becomes in order of the medication dispensing information B, medication dispensing information A, medication dispensing information C, medication dispensing information D, medication dispensing information E, medication dispensing information F, and medication dispensing information G.

Furthermore, when there is no first medication dispensing information including the first medication dispensing information in operation S420, the controller 40 may dispense medications by taking a formal sequence received through the communication unit 30 as the real sequence.

Furthermore, when the first medication dispensing information including the first medication in the medication dispensing information A to G received through the communication unit 30 is in plurality, and the first medication storage unit 14 is connected to the interface unit 20, the controller 40 may dispense the first medications on the basis of any one piece of first medication dispensing information and then sequentially dispense the first medications on the basis of another piece of first medication dispensing information.

In addition, when medication dispensing information additionally received through the communication unit 30 includes the first medication, the controller 40 may dispense the first medications on the basis of the first medication dispensing information and then sequentially dispense the first medications on the basis of the additionally received medication dispensing information.

According to a medication dispensing apparatus, medications are dispensed in order of medication dispensing information such as prescription but the order of the medication dispensing information can be changed to improve a pharmacy's efficiency in medication dispensing and packaging in medication wrapping paper.

In addition, the pharmacy's efficiency can be maximized by prioritizing medication dispensing information including medications stored in a special medication storage unit than other pieces of medication dispensing information among medication dispensing information.

In addition, a standby time taken by medication supplementation can be eliminated by enabling medications to be dispensed based on medication dispensing information even in a case of medication supplementation to the special medication storage unit.

While the invention has been particularly shown and described with reference to preferred embodiments thereof, it will be understood by those skilled in the art that various changes in form and details may be made therein without departing from the spirit and scope of the invention as defined by the appended claims. The preferred embodiments should be considered in descriptive sense only and not for purposes of limitation. Therefore, the scope of the invention is defined not by the detailed description of the invention but by the appended claims, and all differences within the scope will be construed as being included in the present invention.

## Claims

1. A medication dispensing apparatus dispensing a medication from a medication storing unit on a basis of medication dispensing information of which a formal sequence of medication dispensing is determined, the medication dispensing apparatus comprising:
an interface unit connected to a first medication storage unit holding a first medication;
a communication unit receiving the medication dispensing information; and
a controller dispensing a medication in the formal sequence of the medication dispensing information received through the communication unit and when next time medication dispensing information is first medication dispensing information comprising the first medication, taking a sequence of medication dispensing information remaining by omitting the first medication dispensing information as a real sequence of medication dispensing to dispense the medication in the real sequence.

2. The medication dispensing apparatus of claim 1, wherein when the first medication storage unit holding the first medication is connected to the interface unit, the controller changes the real sequence of medication dispensing for prioritizing the first medication dispensing information and dispenses the medication in the changed real sequence.

3. The medication dispensing apparatus of claim 2, wherein when the first medication dispensing information comprises 1-1 medication dispensing information and 1-2 medication dispensing information, the controller dispenses the first medication on a basis of the 1-1 medication dispensing information and then sequentially dispenses the first medication on a basis of the 1-2 medication dispensing information.

4. The medication dispensing apparatus of claim 3, wherein when an amount of the first medication remaining in the first medication storage unit is smaller than that comprised in the 1-2 medication dispensing information, the controller dispenses the medication in a sequence of medication dispensing information remaining after the 1-2 medication dispensing information is omitted from medication dispensing information that medication dispensing is necessary.

5. The medication dispensing apparatus of claim 2, wherein when medication dispensing information additionally received through the communication unit comprises the first medication, the controller dispenses the first medication on the basis of the first medication dispensing information and then sequentially dispenses the first medication on the basis of the additionally received medication dispensing information.

6. The medication dispensing apparatus of claim 1, wherein when the first medication storing unit holding the first medication is connected to the interface unit while the medication is dispensed in the real sequence, the controller makes the first medication dispensing information come directly behind medication dispensing information corresponding to a medication currently dispensed and dispenses the medication.
